# EUROPEAN PATENT APPLICATION

(11) **EP 4 843 542 A1**
(43) Date of publication of application: **25.11.2026**
(21) Application number: 25754533.5
(22) Date of filing: 17.02.2025
(51) Int. Cl.: A61F 2/90, A61F 2/88, A61F 2/86

(54) **LUMEN-ADJUSTABLE STENT**

(30) Priority: 18.02.2024 CN 202410182381
(71) Applicant: Medihealth Welltone Tech (Guangdong) Co., Ltd., Zhongshan, Guangdong 528407 (CN)
(72) Inventor: HU, Meng, Zhongshan, Guangdong 528407 (CN); LIU, Shiyan, Zhongshan, Guangdong 528407 (CN)
(74) Representative: Bayramoglu et al.
(86) International application number: PCT/CN2025/077559
(87) International publication number: WO 2025/171809

(57) **Abstract**

Disclosed is a lumen-adjustable stent. An adjusting member of the stent includes a first end, a second end, and an intermediate segment connecting the first end to the second end. An accessory is connected to a body of the stent, extends naturally relative to the body, and forms a channel allowing the adjusting member to pass through. The stent includes an initial state and an adjustment state. In the initial state, the first end and the second end are close to two ends of the channel, the intermediate segment passes through the channel, and the natural extension length of the intermediate segment is less than the natural extension length of the accessory. In the adjustment state, the restriction of at least one of the first end and the second end on the accessory is relieved.

## Description

### CROSS REFERENCE TO THE RELATED APPLICATIONS

The present application claims priority to Chinese Patent Application No. 202410182381.3, filed with the China National Intellectual Property Administration on February 18, 2024 and entitled "LUMEN-ADJUSTABLE STENT", which is incorporated herein by reference in its entirety.

### FIELD

The present application relates to the field of medical devices, and specifically to a lumen-adjustable stent.

### BACKGROUND

Stents are a commonly used medical device in the current medical field, which can be widely applied to the heart, blood vessels, liver, and the like. Existing stents are often metal stents with thin film materials sutured, wrapped, or coated thereon, where the metal stents provide radial support and anchor the thin film materials. Currently, almost all covered stents serve as artificial blood vessels for unblocking and diverting blood flow. However, the radial dimensions of existing covered stents are all unique. Although multiple specifications exist, once a specification is determined, it is difficult to readjust the stent after implantation.

For example, stents applied to transjugular intrahepatic portosystemic shunt (TIPS) are generally divided into two types: balloon-expandable stents and self-expanding stents. After a balloon-expandable stent is implanted, the stent itself has poor support performance, and the probability of subsequent stent lumen collapse is high. In addition, limited by extraluminal tissue, the effect of repeated balloon dilation is poor, making it difficult to adjust the diameter. However, after a self-expanding stent is implanted in a blood vessel, it is also difficult to adjust the diameter of the stent.

Currently, for TIPS, clinically, after the stent has been implanted for a period of time, at least 24 hours, at least one pressure measurement is required to determine whether there is a need to adjust the stent. When portal vein pressure has decreased but has not yet reached the reference range, it is necessary to increase the diameter of the stent to reduce the portal vein pressure again, which requires that the stent can be adjusted in diameter after implantation. In related technologies, a layer of deformable ductile material is usually added to the outer wall of the stent to compress the stent. After the stent is implanted, the deformable ductile material undergoes tearing deformation through balloon dilation, thereby reducing the constraint on the diameter of the stent and achieving the effect of adjusting the diameter of the stent.

### SUMMARY

The present application provides a lumen-adjustable stent, including a body and an adjusting assembly. The body includes a framework and a covering that covers at least a part of the framework, and the body encloses to form a lumen. The adjusting assembly includes an adjusting member and an accessory, where the adjusting member includes a first end, a second end, and an intermediate segment connecting the first end to the second end; and the accessory is connected to the body, extends naturally relative to the body, and forms a channel allowing the adjusting member to pass through. The stent includes an initial state and an adjustment state; in the initial state, the first end and the second end are near two ends of the channel respectively, the intermediate segment passes through the channel, a natural extension length of the intermediate segment is less than a natural extension length of the accessory between the first end and the second end, and by limiting natural extension of the accessory forming the channel, the body is at least partially compressed circumferentially to adjust a sectional dimension of the lumen; and in the adjustment state, at least one of the first end and the second end relieves a restriction on the accessory, so that circumferential compression of the body is at least partially restored to allow an adjustment of the sectional dimension of the lumen again.

### BRIEF DESCRIPTION OF THE DRAWINGS

The accompanying drawings herein are incorporated in the specification as a part of the specification, showing embodiments that are in accordance with the present application, and used together with the specification to explain the principle of the present application.

To describe the technical solutions in the embodiments of the present application more clearly, the following briefly introduces the accompanying drawings required for describing the embodiments. Apparently, those of ordinary skill in the art may still derive other drawings from these accompanying drawings without creative efforts.
FIG. 1 is a schematic structural diagram of a lumen-adjustable stent in a natural expansion state according to an embodiment of the present application;
FIG. 2 is a schematic structural diagram of a stent in FIG. 1 in an initial state;
FIG. 3 is a schematic structural diagram of an accessory matched with a framework in any example implementation;
FIG. 4 is a schematic structural diagram at an end of an accessory in any example implementation;
FIG. 5a is a schematic structural diagram of an adjusting member in any example implementation;
FIG. 5b and FIG. 5c are schematic diagrams of formation of a first end or a second end of an adjusting member in FIG. 5a;
FIG. 6 is a schematic structural diagram of an accessory matched with a limiting member in any example implementation;
FIG. 7 is a schematic structural diagram of an accessory in any example implementation;
FIG. 8 is a schematic diagram of a stent in a natural expansion state in an embodiment;
FIG. 9 is a schematic diagram of a stent in FIG. 8 in an initial state;
FIG. 10 is a schematic diagram of a stent in FIG. 8 in an adjustment state; and
FIG. 11 is a schematic diagram of a part of a stent in FIG. 8 in an initial state.

### DETAILED DESCRIPTION

To make the objectives, technical solutions, and advantages of the embodiments of the present application clearer, the following clearly and completely describes the technical solutions in the embodiments of the present application with reference to the accompanying drawings in the embodiments of the present application. Apparently, the described embodiments are some but not all of the embodiments of the present application. All other embodiments obtained by those of ordinary skill in the art based on the embodiments of the present application without creative efforts shall fall within the protection scope of the present application.

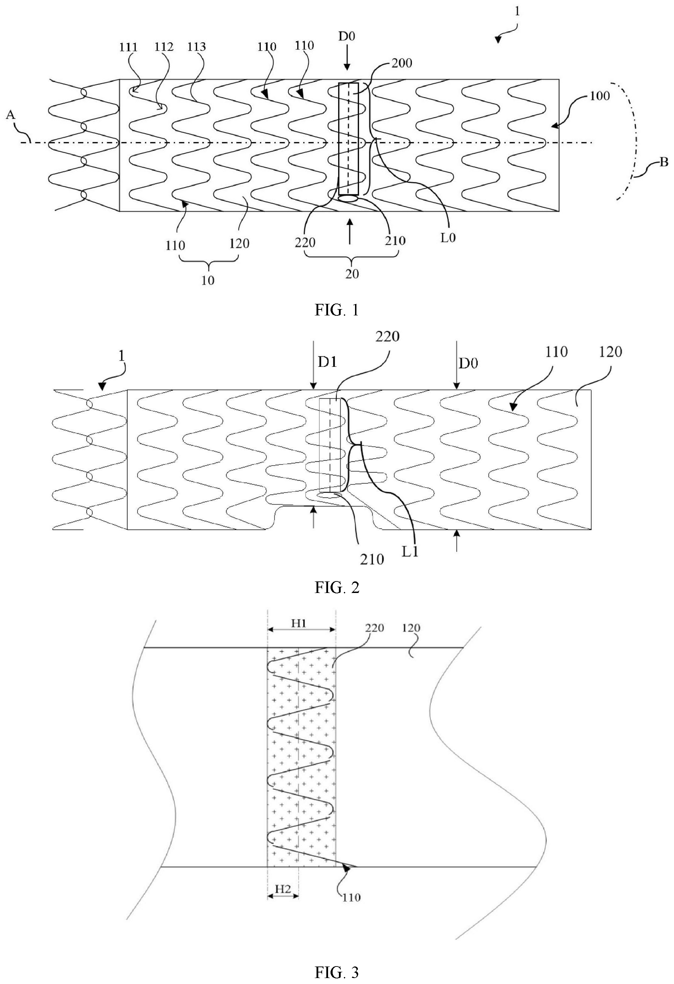

As shown in FIG. 1 and FIG. 2, the present application relates to a lumen-adjustable stent 1, including a body 10 and an adjusting assembly 20. The body 10 includes a framework 110 and a covering 120 that covers at least a part of the framework 110, and the body 10 encloses to form a lumen 100. The adjusting assembly 20 includes an adjusting member 210 and an accessory 220. The adjusting member 210 includes a first end 211, a second end 212, and an intermediate segment 213 connecting the first end 211 to the second end 212. The accessory 220 is connected to the body 10, extends naturally relative to the body 10, and forms a channel 200 allowing the adjusting member 210 to pass through. The stent 1 includes an initial state and an adjustment state. In the initial state, the first end 211 and the second end 212 are near two ends of the channel 200 respectively, the intermediate segment 213 passes through the channel 200, a natural extension length of the intermediate segment 213 is less than a natural extension length of the accessory 220 between the first end 211 and the second end 212, and by limiting natural extension of the accessory 220 forming the channel 200, the body 10 is at least partially compressed in a circumferential direction B to adjust a sectional dimension of the lumen 100. In the adjustment state, at least one of the first end 211 and the second end 212 relieves a restriction on the accessory 220, so that compression of the body 10 in the circumferential direction B is at least partially restored to allow an adjustment of the sectional dimension of the lumen 100 again.

The lumen-adjustable stent 1 of the present application (hereinafter referred to as the stent 1 for short) changes the extension length of the accessory 220 through the adjusting member 210, thereby changing a form in the circumferential direction B of the body 10 connected to the accessory 220, so as to adjust a radial dimension of the lumen 100 formed by the body 10, and finally achieve an effect of adjusting the diameter of the stent 1. By using this adjustment method, the diameter of the stent 1 can be adjusted only by changing the restriction or constraint of the adjusting member 210 relative to the accessory 220. Therefore, the integrity of the body 10 of the stent 1 during diameter adjustment of the lumen 100 may be ensured, without affecting the strength of the stent 1, for example, causing an uneven strength distribution of the stent 1, thereby preventing tissue from penetrating a surface of the stent 1 and growing into the lumen 100 of the stent 1 after the stent 1 is implanted, or preventing tissue overgrowth from squeezing the lumen 100 of the stent 1, so that the safety and reliability of the stent 1 after surgery are improved.

Specifically, referring to FIG. 1, the stent 1 has a natural expansion state. In the natural expansion state, both the first end 211 and the second end 212 do not limit the accessory 220, or have completely relieved the restriction on the accessory 220. The accessory 220 naturally extends, and at this time, the natural extension length thereof is L0. It may be understood that to implement that the accessory 220 changes a radial compression form of the body 10 of the stent 1, the accessory 220 may be prevented from naturally extending in a direction parallel to an axial direction A, ensuring that the accessory at least partially or fully extends naturally in the circumferential direction B of the body 10. In some implementations, the accessory may be connected to the body 10 on an outer peripheral surface of the body 10 and extend in the circumferential direction B. The circumferential direction B may be considered as an outer circumferential direction of the body 10. The accessory 220 extending in the circumferential direction B may be understood as the accessory extending directly in the circumferential direction B, or may be understood as a projection of the accessory on a cross-section perpendicular to the axial direction A extending in the circumferential direction B. Therefore, when the accessory 220 is in the natural expansion state, the body 10 at the connection of the accessory 220 is not compressed or limited, and the diameter of the lumen 100 is a naturally expanded dimension D0.

Referring to FIG. 2, the stent 1 further has the initial state. In the initial state, the first end 211 and the second end 212 of the adjusting member 210 are near the two ends of the channel 200 respectively, and the intermediate segment 213 passes through the channel 200. For example, dimensions of both the first end 211 and the second end 212 may be configured to be greater than an internal dimension of the channel 200. When the first end 211 and/or the second end 212 are disposed near the end outside the channel 200 or near the end inside the channel 200, due to the dimensions, the first end 211 and the second end 212 will be clamped at the ends of the channel 200 formed by the accessory 220, so that the adjusting member 210 is temporarily clamped in the accessory 220. In addition, the natural extension length of the intermediate segment 213 is configured to be less than the natural extension length of the accessory 220 between the first end 211 and the second end 212. Because the first end 211 and the second end 212 of the adjusting member 210 have been clamped, the length of the accessory 220 between the first end 211 and the second end 212 will be limited or constrained to be close to the length of the intermediate segment 213, limiting the natural extension of the accessory 220 forming the channel 200, so that the extension length of the accessory 220 is controlled and adjusted by the length of the intermediate segment 213. Therefore, the extension length of the accessory 220 is compressed from the natural extension length L0 to L1, where L1 < L0. The compression of the accessory 220 causes the body 10 connected to the accessory 220 to be compressed simultaneously, thereby changing the diameter of the body 10 at the connection of the accessory 220, which is compressed from the naturally expanded D0 to D1, where D1 < D0, implementing the adjustment of the sectional dimension of the lumen 100.

The stent 1 further has the adjustment state. In the adjustment state, at least one of the first end 211 and the second end 212 relieves the restriction on the accessory 220, so that the compression of the body 10 in the circumferential direction B is at least partially restored. For example, after the first end 211 relieves the restriction on the accessory 220, the accessory 220 on one side of the first end 211 relieves a compressed or constrained state and may gradually restore the length. At this time, the accessory 220 has an adjusted extension length L2, where L1 < L2 < L0. In this way, the compression of the body 10 in the circumferential direction B at the connection of the accessory 220 is also correspondingly relieved gradually, and an adjusted diameter D2 thereof meets D1 < D2 < D0, to adjust the sectional dimension of the lumen 100 again.

Based on this, it can be seen that in the stent 1 of the present application, by configuring constraint matching between the adjusting member 210 and the accessory 220, the extension length of the accessory 220 in the circumferential direction B is changed, thereby changing a compression shape of the body 10 in the circumferential direction B at the connection of the accessory 220, to implement the adjustment of the diameter of the stent 1. This adjustment does not affect the structure of the body 10 itself, and therefore does not affect the strength of the stent 1 after implantation, ensuring the implantation safety of the stent 1. At the same time, tissue growth at positions with weak strength of the stent 1 may also be avoided, thereby preventing the tissue growth from squeezing the lumen 100 of the stent 1, causing stenosis and occlusion. In addition, the above adjustment is controllable. At least one of the first end 211 and the second end 212 may be controlled to relieve the restriction or constraint, and the gradual relieving of the restriction or constraint of the first end 211 and/or the second end 212 within the channel 200 of the accessory 220 may further be controlled. Compared with the related technologies, which usually can only adjust the diameter of the stent 1 by tearing a deformable ductile material attached to an outer surface of the stent 1, the stent 1 of the present application has higher adjustment precision and more adjustable dimensions within a diameter adjustment range thereof.

Specifically, the body 10 of the stent 1 of the present application is of a tubular structure with openings at both ends and a closed middle, and the formed lumen 100 constitutes a blood flow channel for blood circulation. The framework 110 thereof includes at least one annular wave ring. When there are a plurality of annular wave rings, the plurality of annular wave rings are disposed at intervals in the axial direction. An "annulus" of the annular wave ring may be a closed annulus or an open annulus extending helically in the axial direction. The plurality of annular wave rings may be connected, for example, flexibly connected or rigidly connected, or may not be connected, which is not described herein in detail again. The annular wave ring further includes a plurality of alternating and staggered peaks 111 and troughs 112, and a plurality of connecting rods 113 connecting adjacent peaks 111 and troughs 112. A waveform in the annular wave ring may be a sine wave, a square wave, a triangular wave, or the like, which is not enumerated herein again. This waveform structure of the annular wave ring is prone to compression and expansion, which helps improve the ductility of the overall structure of the framework 110.

The framework 110 may be made of a material having good biocompatibility and good elasticity, such as stainless steel, a nickel-titanium alloy or cobalt-chromium alloy, and an iron-based material. In an actual manufacturing process, the framework 110 is formed by braiding and setting a nickel-titanium wire or cutting and setting a nickel-titanium tube. Certainly, the framework 110 may also be formed by braiding or cutting and setting a stainless steel wire. By selecting the material for the framework 110, a self-expanding framework 110 or an axially expanding framework 110 may be formed. It should be understood that the above framework 110 is only used as an example and is not intended to limit the present application. Those of ordinary skill in the art can select a suitable framework 110 according to usage scenarios and actual requirements, which is not described herein in detail again.

In some implementations, the framework 110 may have a self-expanding force, that is, the framework 110 tends to restore to a natural expansion form thereof when subjected to an external constraint force. When the external constraint force applied to the framework 110 is greater than the current self-expanding force of the framework 110, the framework 110 is continuously compressed at a position where the external constraint force is applied. When the framework 110 is continuously compressed until the self-expanding force is equal to the external constraint force, the framework 110 remains in a compressed state. When the external constraint force applied to the framework 110 decreases, the framework 110 gradually expands under the action of the self-expanding force thereof. When the external constraint force disappears, the framework 110 restores to a natural expansion state thereof.

The covering 120 may be made of a thin film material with good biocompatibility, such as polyethylene terephthalate (referred to as PET for short) and expanded polytetrafluoroethylene (referred to as e-PTFE for short). By disposing the covering 120 and the framework 110, the tubular structure with openings at both ends and a closed middle is formed, constituting the blood flow channel for blood or other fluids to pass through. The lumen 100 of the body 10 constitutes the blood flow channel, and a cross-section of the lumen 100 may be of a circular shape, an elliptical shape, or other shapes.

The accessory 220 of the adjusting assembly 20 is connected to the body 10 on the outer peripheral surface of the body 10 and forms the channel 200 allowing the adjusting member 210 to pass through, and at least a part of the accessory 220 on the outer peripheral surface of the body 10 extends in the circumferential direction B. This may be understood as extending directly in the circumferential direction B, or as the projection of the accessory on the cross-section perpendicular to the axial direction A extending in the circumferential direction B, to prevent the accessory from parallelly extending in the axial direction A. For example, the accessory 220 may be strip-shaped. Two long sides thereof are connected to the body 10 respectively to form the channel 200; and two short sides thereof form the two ends of the channel 200 respectively. Certainly, in addition to a strip-shaped square shape, any other shape is permissible, as long as at least a part of the accessory extends in the circumferential direction B on the outer peripheral surface of the body 10, which is not enumerated herein again. In addition, the two ends of the channel 200 may be configured to be closed relative to the body 10, or may be configured to have openings relative to the body 10. At the same time, open and closed states of the two ends may be arbitrarily combined.

It may be understood that the accessory 220 constrains the body 10 in the circumferential direction B, and the effect exhibited is that the length in the circumferential direction B and the radial dimension of the body 10 are simultaneously reduced. That is, the effect of the accessory 220 constraining the body 10 in the circumferential direction B is equivalent to the accessory 220 constraining the body 10 in a radial direction. Similarly, when the constraint of the accessory 220 on the body 10 in the circumferential direction B disappears, the effect thereof is equivalent to the disappearance of the constraint of the accessory 220 on the body 10 in the radial direction. In other words, after the accessory 220 in the present application constrains the body 10 in the circumferential direction B, the radial dimension of the body 10 also becomes smaller accordingly. Therefore, the accessory 220 in the present application constrains the body 10 in the circumferential direction B, thereby achieving the effect of constraining the body 10 in the radial direction.

The accessory 220 may be made of a material having good biocompatibility and deformability under stress, such as a flexible material, stainless steel, a platinum-iridium alloy, an iron-based material, and gold. The flexible material is preferred, for example, PTFE, PET, or PP. In different usage scenarios, materials with different elongation rates may be selected. For example, a material with a smaller elongation rate may be used to increase a constraint force on the covering 120 and the framework 110 in the circumferential direction B, thereby enhancing a radial shape retention capability of the stent 1. Alternatively, a material with a larger elongation rate may be used to reduce a constraint force on the covering 120 and the framework 110 in the circumferential direction B. In some implementations, the accessory 220 may be made of a material the same as that of the covering 120 to ensure compression consistency.

Referring to FIG. 3, an axial distance between the peak 111 and the trough 112 in the annular wave ring may be defined as a wave height H1, and a region where H1 is located is an elastic region of the annular wave ring. At the same time, an axial length of the annular wave ring constrained by the accessory 220 is defined as H2, where H2 is greater than or equal to half of H1, that is, H2 ≥ 1/2H1. At this time, a region where the accessory 220 constrains the annular wave ring is the elastic region, that is, the accessory 220 may completely constrain the elastic region to constrain the body 10 in the circumferential direction B, thereby reducing the diameter of the body 10. Therefore, the elastic region on the annular wave ring may be completely constrained, which can prevent instability in the connection of the accessory 220 caused by unevenness on both sides of the annular wave ring (one side is higher, and the other side is lower), thereby helping improve the constraint effect of the accessory 220 on the body 10.

Different accessories 220 are selected, which determines a constraint restriction manner between the adjusting member 210 and the accessory 220 and a corresponding constraint restriction relieving manner. That is, adjusting the dimension of the channel 200 may constrain and restrict the adjusting member 210, and opening the channel 200 or increasing the dimension thereof may relieve the constraint restriction. For example, when the accessory 220 is made of a material such as an ePTFE film or FEP, the channel 200 may be enlarged through the principle of hot-press separation of two layers of films. When the accessory 220 is made of an elastic material (such as silicone) that can increase in area, the channel 200 may be enlarged by deforming the accessory 220. When the accessory 220 is made of a material such as PET by suturing through connection, the channel 200 may be enlarged in manners such as enabling the accessory 220 to rupture or enabling a suture position of the accessory 220 to fail. The manner of enabling the suture position to fail may be suture breakage, suture elastic elongation, suturing knot drop-off, suturing knot loosening, suturing knot deformation, and the like. When the accessory 220 is made of a human-absorbable material, the human body absorbs and degrades the accessory 220 at the connection, and after a period of time, the accessory 220 gradually disappears, to relieve the constraint on the adjusting member 210. A plurality of channel opening manners above are only for illustration and are not intended to limit the present application. Based on the teachings of the present application, other manners may further be used to open the channel 200, which is not described herein in detail again.

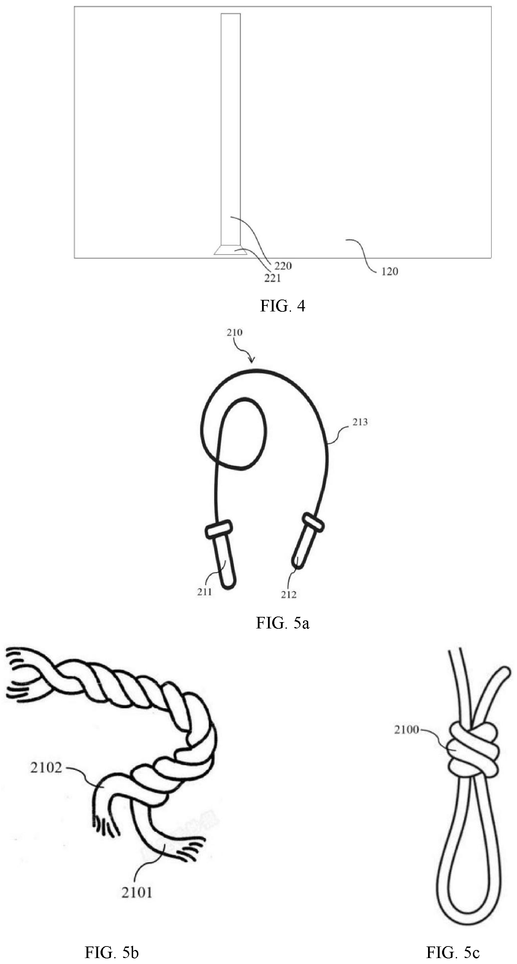

Further, referring to FIG. 4, a sectional area of a part of the accessory 220 close to an end 221 is greater than a sectional area of a remaining part (or a sectional area of a part of the accessory 220 close to an end 221 gradually increases). The end 221 may be of an opening structure to facilitate the entry of the first end 211 or the second end 212 of the adjusting member 210 into the channel 200. Under the action of an external force, for example, when a balloon expands, the adjusting member 210 may precisely expand the connection between the accessory 220 and the covering 120, so that the adjusting member 210 moves in the channel 200 toward one side away from the end 221, to relieve the radial constraint of the accessory 220 on the body 10.

The stent 1 may include at least two accessories 220 that are relatively staggered from each other. The at least two accessories 220 may be arranged at intervals in the circumferential direction B of the body 10. At this time, the at least two accessories 220 may perform multi-segment constraint on the body 10 in the circumferential direction B according to actual needs, which helps improve the constraint effect. In addition, the at least two accessories 220 may also be arranged at intervals in the axial direction of the body 10. At this time, the at least two accessories 220 can perform multi-segment constraint on the body 10 in the axial direction according to actual needs, which helps further improve the constraint effect. In some implementations, when the accessory 220 is long enough, the accessory 220 can constrain the body 10 in the circumferential direction B. This manner can constrain the body 10 more uniformly in the circumferential direction B, which helps adjust the diameter of the body 10 subsequently. Certainly, one accessory 220 may further wrap around the body 10 at least one turn in the circumferential direction B, to perform one or more turns of constraint on the body 10 in the circumferential direction B. In this manner, the body 10 is constrained more firmly, and the risk of constraint failure is lower.

Referring to FIG. 5a, in the stent 1 of the present application, the intermediate segment 213 of the adjusting member 210 is a flexible rope. At least one of the first end 211 and the second end 212 is an elastic deformation body or a plastic deformation body and is connected to the intermediate segment 213 on two opposite sides of the intermediate segment 213 respectively. For example, at least one of the first end 211 and the second end 212 is spherical, olive-shaped, conical, or profiled, and may also be formed into a final shape after being braided and set.

In some implementations, in combination with FIG. 5b and FIG. 5c, the intermediate segment 213 of the adjusting member 210 is a cable. At least one of the first end 211 and the second end 212 is an elastic deformation body 2100 formed by knotting a cable braided from a mixture of a PET yarn 2101 and a PTFE yarn 2102. Therefore, the constraint restriction can be relieved not only by opening the channel 200 of the accessory 220 through material selection of the accessory 220, but also by elastic deformation of at least one of the first end 211 and the second end 212, or by the simultaneous action of the accessory 220 and the adjusting member 210.

In some implementations, a dimension of the first end 211 is greater than a dimension of an adjacent end of the channel 200, to limit the natural extension of the accessory 220 on this side by clamping the first end 211 at the end. A dimension of the second end 212 is greater than a dimension of an adjacent end of the channel 200, to limit the natural extension of the accessory 220 on this side by clamping the second end 212 at the end. Under the action of an external force, such as balloon expansion, the accessory 220 opens the channel 200 through at least one of the above multiple methods, for example, through elastic deformation or plastic deformation of the accessory 220, the first end 211 and/or the second end 212 enter the channel 200, thereby relieving the constraint restriction relative to the accessory 220; or the first end 211 and/or the second end 212 enter the channel 200 through self-deformation, elastic deformation, or plastic deformation to relieve the constraint restriction relative to the accessory 220; or the accessory 220 and the first end 211 and/or the second end 212 of the adjusting member 210 work together to relieve the constraint restriction.

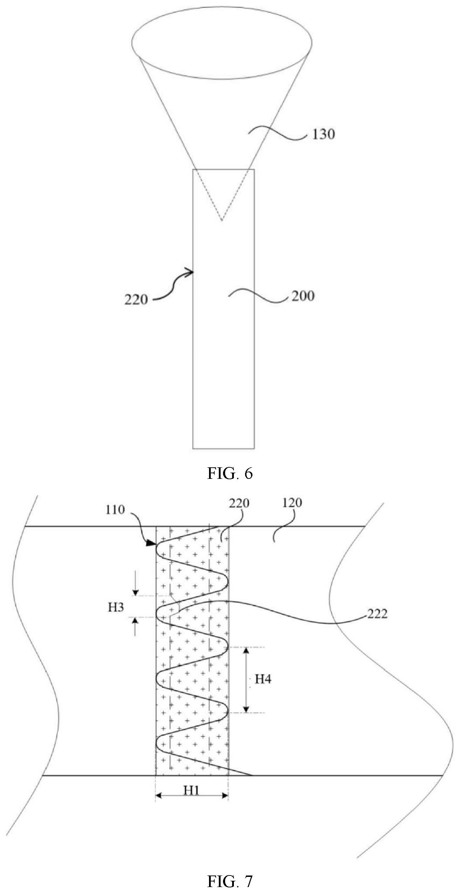

Referring to FIG. 6, in another implementation of the present application, the body 10 further includes a limiting member 130. The limiting member 130 is of a structure with one large end and one small end, for example, a frustum-shaped structure, a drop-shaped structure, a ball-shaped structure, or an annular body structure. A part of the limiting member 130 is inserted into the end of the accessory 220, and an inner cavity of the limiting member 130 is communicated with the channel 200. A bottom side of the limiting member 130 having a smaller caliber extends into the channel 200 from the end of the channel 200, and a bottom end side of the limiting member 130 having a larger caliber is outside the channel 200 and clamped at the end of the channel 200. The adjusting member 210 passes through the limiting member 130 into the channel 200, to limit the natural extension of the accessory 220 on this side by clamping at least one of the first end 211 and the second end 212 in the inner cavity of the limiting member 130. By using the limiting member 130, under the action of an external force, it may be effectively ensured that the first end 211 or the second end 212 relieves the constraint restriction and enters the channel 200, thereby improving the effectiveness and accuracy of diameter adjustment.

In some implementations, the limiting member 130 is a mesh body and covers the end of the channel 200. The mesh body includes at least one mesh hole, and a natural aperture dimension of the mesh hole is simultaneously less than dimensions of the first end 211 and the second end 212, to limit the natural extension of the accessory 220 on this side by clamping at least one of the first end 211 and the second end 212 at the mesh hole of the limiting member 130. Under the action of an external force, at least one of the first end 211 and the second end 212 breaks through the restriction of the mesh hole and enters the channel 200 through deformation, for example, elastic deformation or plastic deformation, thereby relieving the constraint restriction relative to the accessory 220; or under the action of an external force, a mesh cable forming the mesh hole in the limiting member 130 deforms or breaks, and the first end 211 or the second end 212 breaks through the restriction of the mesh hole and enters the channel 200, thereby relieving the constraint restriction relative to the accessory 220; or the mesh cable and the first end 211 or the second end 212 work together. By using the limiting member 130, under the action of an external force, it may be effectively ensured that the first end 211 or the second end 212 relieves the constraint restriction and enters the channel 200, thereby improving the effectiveness of diameter adjustment.

From the above examples, it can be seen that after the first end 211 or the second end 212 relieves the constraint restriction relative to the accessory 220, at least one of the first end 211 and the second end 212 may continue to travel in the channel 200 under the action of an external expansion force, to further relieve the restriction on the accessory 220 until the accessory 220 restores to the natural extension length, thereby precisely adjusting the diameter of the stent 1 within a certain range, rather than adjusting the diameter of the stent 1 at one time.

Referring to FIG. 7, in an example implementation of the present application, the accessory 220 may extend on the body to form a bending constraint portion 222. The bending constraint portion 222 is connected to the covering 120. An axial distance between the peak 111 and the trough 112 in the annular wave ring is defined as a wave height H1, a region where H1 is located is an elastic region of the annular wave ring, a wavelength of the annular wave ring is H4, and a length of the bending constraint portion 222 in the circumferential direction B is H3. At this time, H3 < 1/2H4. The bending constraint portion 222 can increase the friction between the accessory 220 and the body 10, reducing the impact of external friction on the stent 1 during transportation, assembly, and release, which helps avoid the occurrence of a large separation force at the connection position between the accessory 220 and the covering 120, preventing the accessory 220 from failing in advance.

It can be further understood that there may be more than one bending constraint portion 222. Disposing a plurality of bending constraint portions 222 can further improve the connection firmness between the accessory 220 and the body 10, preventing the accessory 220 from failing in advance.

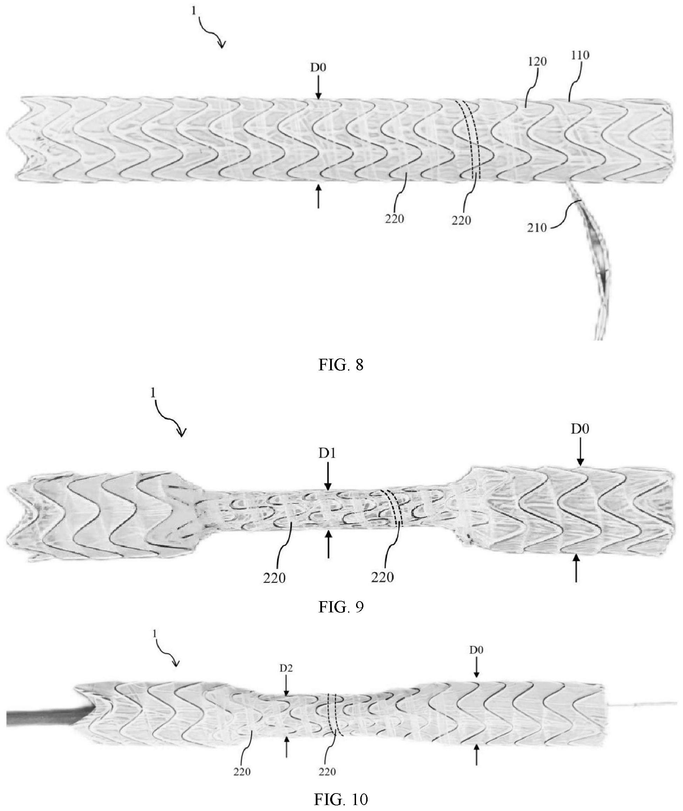

Referring to FIG. 8 to FIG. 15, in an embodiment of the present application, the accessory 220 is helically wound on the outer peripheral surface of the body 10 for a plurality of turns, that is, the accessory extends and covers a plurality of turns in the circumferential direction B. A dashed line in the figures schematically shows a segment of the accessory, so that the body 10 may be uniformly compressed in the circumferential direction B over a certain axial length. The end of the accessory 220 is provided with a frustum-shaped limiting member 130. Both the first end 211 and the second end 212 of the adjusting member 210 are elastic deformation bodies formed by knotting a cable braided from a mixture of a PET yarn and a PTFE yarn. The intermediate segment 213 of the adjusting member 210 passes through the channel 200 formed by the accessory 220. The limiting member 130 at the end of the accessory 220 limits the first end 211 or the second end 212 from entering the channel 200, thereby implementing the constraint restriction of the adjusting member 210 on the accessory 220.

Referring to FIG. 8 and FIG. 9, the stent 1 is in the natural expansion state, with a diameter of D0.

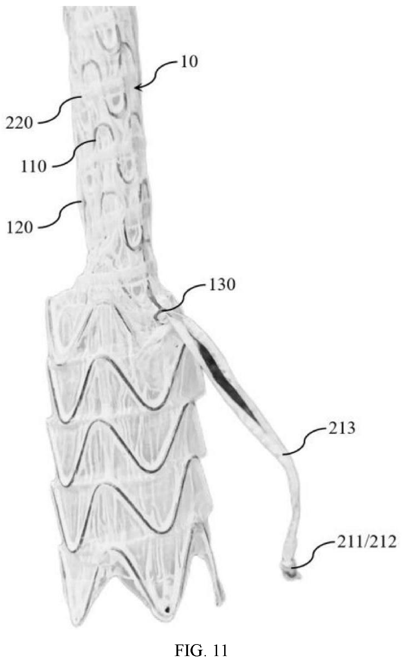

Referring to FIG. 10 and FIG. 11, the stent 1 is in the initial state, and the accessory 220 is constrained by the first end 211 and the second end 212, thereby radially compressing the body. At this time, the diameter of the stent 1 is D1.

Referring to FIG. 12 and FIG. 13, under the action of an external force, for example, balloon expansion, the first end 211 or the second end 212 undergoes elastic deformation and is squeezed into the channel 200, thereby at least partially relieving the constraint on the accessory 220, and then at least partially relieving the constraint on the body 10. The stent is in the adjustment state, with a diameter of D2, where D1 < D2 < D0. Since an original dimension of the first end 211 or the second end 212 is greater than that inside the channel 200, even if the first end 211 or the second end 212 enters the channel 200, the first end or the second end may be stuck in the channel 200. If an external force is further applied, for example, continuing the balloon expansion, on one hand, the first end 211 or the second end 212 may further deform, and it is also possible that the accessory 220 may undergo deformation, tearing, or expansion, such as increased deformation, tearing away from the body 10, or expanding relative to the body 10, so that the first end 211 or the second end 212 may continue to move in the channel 200, thereby further relieving the constraint restriction on the accessory 220 and finally implementing continuous and progressive adjustment of the diameter of the body 10. It should be noted that, during the above deformation, tearing, or expansion of the accessory 220, the structure of the body 10 remains unchanged.

It should be noted that relational terms such as "first" and "second" are used herein merely to distinguish one entity or operation from another entity or operation, and do not necessarily require or imply any such actual relationship or order between these entities or operations. Moreover, the terms "include", "comprise", or any other variants thereof are intended to cover non-exclusive inclusion, so that a process, method, item, or device including a series of elements includes not only those elements but also other elements not explicitly listed, or further includes inherent elements of such process, method, item, or device. An element defined by "includes a..." does not, without more constraints, preclude the existence of additional identical elements in the process, method, item, or device that includes the element.

The foregoing descriptions are merely detailed descriptions of the present application, so that those skilled in the art can understand or implement the present application. Various modifications to these embodiments will be readily apparent to those skilled in the art, and the general principles defined herein may be implemented in other embodiments without departing from the spirit or scope of the present application. Therefore, the present application will not be limited to the embodiments shown herein, but is to be in accordance with the widest scope consistent with the principles and novel features disclosed herein.

## Claims

1. A lumen-adjustable stent, comprising:
a body, comprising a framework and a covering that covers at least a part of the framework, and enclosing to form a lumen; and
an adjusting assembly, comprising an adjusting member and an accessory, wherein the adjusting member comprises a first end, a second end, and an intermediate segment connecting the first end to the second end; and the accessory is connected to the body, extends naturally relative to the body, and forms a channel allowing the adjusting member to pass through,
wherein the stent comprises an initial state and an adjustment state; in the initial state, the first end and the second end are near two ends of the channel respectively, the intermediate segment passes through the channel, a natural extension length of the intermediate segment is less than a natural extension length of the accessory between the first end and the second end, and by limiting natural extension of the accessory forming the channel, the body is at least partially compressed circumferentially to adjust a sectional dimension of the lumen; and in the adjustment state, at least one of the first end and the second end relieves a restriction on the accessory, so that circumferential compression of the body is at least partially restored to allow an adjustment of the sectional dimension of the lumen again.

2. The lumen-adjustable stent according to claim 1, wherein the stent further comprises a natural expansion state; and in the natural expansion state, both the first end and the second end relieve the restriction on the accessory, and the accessory extends naturally.

3. The lumen-adjustable stent according to claim 1 or 2, wherein the accessory is strip-shaped, two long sides thereof are connected to the body respectively to form the channel, and two short sides thereof form the two ends of the channel respectively.

4. The lumen-adjustable stent according to any one of claims 1 to 3, wherein the intermediate segment of the adjusting member is a flexible rope; and at least one of the first end and the second end is an elastic deformation body or a plastic deformation body and is connected to the intermediate segment on two opposite sides of the intermediate segment respectively.

5. The lumen-adjustable stent according to any one of claims 1 to 4, wherein the intermediate segment of the adjusting member is a cable; and at least one of the first end and the second end is an elastic deformation body formed by knotting a cable braided from a mixture of a polyethylene terephthalate (PET) yarn and a polytetrafluoroethylene (PTFE) yarn.

6. The lumen-adjustable stent according to any one of claims 1 to 5, wherein a dimension of the first end is greater than a dimension of an adjacent end of the channel, to limit the natural extension of the accessory by clamping the first end at the end; and a dimension of the second end is greater than a dimension of an adjacent end of the channel, to limit the natural extension of the accessory by clamping the second end at the end.

7. The lumen-adjustable stent according to any one of claims 1 to 6, wherein the body further comprises a limiting member, the limiting member is of a frustum-shaped structure, a part of the limiting member is inserted into the end of the accessory, and an inner cavity of the limiting member is communicated with the channel, wherein a bottom side of the limiting member having a smaller caliber extends into the channel from the end of the channel, and a bottom end side of the limiting member having a larger caliber is outside the channel and clamped at the end of the channel; and the adjusting member passes through the limiting member into the channel, to limit the natural extension of the accessory by clamping at least one of the first end and the second end in the inner cavity of the limiting member.

8. The lumen-adjustable stent according to any one of claims 1 to 7, wherein the body further comprises a limiting member, and the limiting member is a mesh body and covers the end of the channel; and the mesh body comprises at least one mesh hole, and a natural aperture dimension of the mesh hole is simultaneously less than dimensions of the first end and the second end, to limit the natural extension of the accessory by clamping at least one of the first end and the second end at the mesh hole of the limiting member.

9. The lumen-adjustable stent according to any one of claims 1 to 8, wherein when an external expansion force is applied to the body within the lumen, at least one of the first end and the second end enters the channel by tearing the accessory to relieve the restriction on the accessory; at least one of the first end and the second end enters the channel through elastic deformation of the at least one of the first end and the second end and/or the accessory to relieve the restriction on the accessory; or at least one of the first end and the second end enters the channel through plastic deformation of the at least one of the first end and the second end and/or the accessory to relieve the restriction on the accessory.

10. The lumen-adjustable stent according to claim 9, wherein at least one of the first end and the second end passes through the channel under an action of the external expansion force, to further relieve the restriction on the accessory.

11. The lumen-adjustable stent according to any one of claims 1 to 10, wherein the accessory extends on the body to form a bending constraint portion.
